(19) Europäisches Patentamt
European Patent Office
Office européen des brevets

(11) Publication number: 0 260 079 A1

(12) **EUROPEAN PATENT APPLICATION**

(21) Application number: 87307841.4

(22) Date of filing: 04.09.87

(51) Int. Cl.[4]: G 01 N 33/546
G 01 N 33/563

(30) Priority: 05.09.86 GB 8621495

(43) Date of publication of application:
16.03.88 Bulletin 88/11

(84) Designated Contracting States:
BE CH DE FR GB IT LI LU NL SE

(71) Applicant: Acade Diagnostic Systems SA/NV
Passage de la Vecquee 17 Box 4100
B-1200 Brussels (BE)

(72) Inventor: Saint-Remy, Jean-Marie
Rue de Lambais, 79
B-5980 Grez-Doiceau (BE)

Mareschal, Jean-Claude
Avenue de la Vecquee 196
B-5730 Malonne (BE)

Gilles, Jean Guy
410/Bt 51, Ave de l'Exposition
B-1090 Brussels (BE)

Masson, Pierre Lucien
Avenue E. Vandervelde
B-1200 Brussels (BE)

Wilkins, Terence Arthur
Vosberg 20
B-1970 Wezembeek - Oppem (BE)

(54) **Method of assay.**

(57) In an assay for an analyte, a sample of biological fluid is first incubated with first insoluble particles carrying a specific binder for the analyte. The supernatant liquid, containing potentially interferring species, is discarded. The analyte/specific binder complex is treated, e.g. with enzyme, to re-solubilize the analyte or an antigenically distinctive fragment thereof. To the resulting assay fluid, second insoluble particles are added and their agglutination is measured, either by turbidimetry or by particle counting. The method is particularly suitable for identifying IgE antibodies directed against specific allergens in serum or plasma.

EP 0 260 079 A1

**Description**

## METHOD OF ASSAY

This invention is concerned with a method of assaying for an analyte in a biological fluid. Such assays frequently suffer from interference by other species present in the biological fluid. One way of overcoming such interference is to perform a preliminary step in which the analyte is separated from the remainder of the biological fluid prior to assay. The present invention is concerned with a method of this kind.

USP 3720760 relates to an in vitro method of analysing a body fluid for antibodies to allergens. A sample of the body fluid is contacted with the allergens which have been previously immobilized on insoluble particles. The particles are separated and washed, and are then reacted with a labelled reagent, allergen or antibody. The extent of binding of label to the insoluble particles indicates the concentration of antibody in the original sample.

GB 2030290 describes a method for assaying free analyte, such as thyroid hormone, in a biological sample containing the analyte in both free and bound forms. In a first step, a sample of the fluid is contacted with an unlabelled receptor for the analyte, which has been insolubilised as a coating on the walls of the assay tube, so as to cause free analyte to bind to the receptor. After removal of the supernatent liquid and washing, the tube is then contacted with a solution of either the labelled analyte or a labelled receptor. The extent of binding of label to the tube indicates the free analyte concentration in the original fluid.

A disadvantage of these prior art methods is that the second incubation is performed with the analyte in an insolubilised state. This certainly makes the analyte physically less readily available for reaction; it may also decrease its reactivity or alter its properties.

European Patent 51985 describes a test for a specific IgE in which a sample is contacted with an absorbent disc carrying an antigen (allergen) to the IgE. The disc is then contacted with a protease to digest the bound IgE, producing an antigenically distinctive fragment which is then assayed. A disadvantage of this procedure is that, the surface of the disc being small, competition with antibodies of the same specificity but other isotypes can result in underestimation of the titre of the specific IgE.

The present invention provides a method of assaying for an analyte in a biological fluid, which method comprises the steps of:-

a) contacting a sample of the biological fluid with first particles carrying a specific binder for the analyte under conditions to cause analyte to bind to the specific binder, and recovering the first particles carrying an analyte/specific binder complex,

b) treating the first particles carrying the complex to bring into solution the analyte or an antigenically distinctive fragment thereof,

c) adding second particles to the solution to form an assay fluid under conditions to effect agglutination of the second particles, and determining the extent of such agglutination as an assay of the analyte in the sample.

As regards the nature of the analyte, the invention is generally applicable. The analyte may be an antigen or a hapten, in which case the specific binder will generally, but need not necessarily, be its corresponding antibody. Or the analyte may be an antibody, in which case, the specific binder may conveniently be its associated antigen or a second antibody thereto. The invention is applicable to qualitative and quantitative assays. When applied quantitatively, it is applicable to the assay of total analyte concentration alternatively of free analyte concentration. Examples of the use of the method in the assay of total and free analytes are given below. Similarly, the nature of the biological fluid is not at all important to the invention.

The first step of the method involves forming a reaction mixture by contacting a sample of the biological fluid with first particles carrying a specific binder for the analyte. The mixture is incubated under known conditions suitable to cause the analyte to bind to the specific binder. The specific binder may be insolubilised by any of various known techniques. For example, it may be covalently attached to small insoluble particles, e.g. of polystyrene, which are suspended in the reaction medium. For ease of separation from the reaction medium, the particles may be made magnetically attractable.

For a total analyte assay and to ensure that most or all of the analyte becomes bound to the specific binder, it is generally preferable to use an amount of specific binder which is in excess over the expected amount of analyte in the sample. On the other hand, where a free analyte concentration is being assayed, it is generally desirable that the specific binder be not used in a concentration so high as to substantially affect the equilibrium between free analyte and analyte bound to natural protein binders. While the absolute amount of specific binder may not be critical, it is generally important that the amount be accurately the same from one assay tube to the next. This enables a calibration curve to be determined by the use of standards, from which the analyte concentrations of unknown samples can be subsequently be read off.

The insolubilised analyte/specific binder complex is separated from the remainder of the reaction mixture by any suitable means, e.g. centrifuging, magnetic attraction or simple aspiration of the supernatant liquid. Preferably, the insolubilised complex is washed to ensure complete removal of unattached reagents. It is then ready for treatment by the second step of the method of the invention, to bring the analyte, or an antigenically distinctive fragment thereof, back into solution. In most cases, this treatment may conveniently be effected by means of an enzyme such as pepsin, or alternatively trypsin. For example, where an insolubilised antibody has been used in step a) to capture a non-protein antigen or hapten, treatment with pepsin can be used to destroy the antibody and release the antigen or hapten into solution.

In the case where an insolubilised antigen or hapten has been used in step a) to capture an antibody, controlled enzyme action can be used to digest the antibody and release Fc fragments thereof into solution. An advantage of this procedure is that some of the Fc fragments are known to be very highly antigenic, and to be capable of being assayed with much greater sensitivity than is the whole antibody. When the assay is performed by an agglutination technique (see below), these fragments have the further advantage of forming more stable agglutinates than intact antibodies, thus further enhancing robustness and sensitivity.

Where an insolubilised antibody has been used in step a), it may be possible to use a reagent other than an enzyme to unfold the antibody and thus release the analyte into solution. An example of such a reagent is guanidinium hydrochloride used at a concentration of 3 to 6 mol/l. The nature and extent of the treatment necessary to bring the analyte (or a fragment thereof, as noted above) into solution is either known or determinable by routine experiment.

Having been brought into solution in the absence of interferring species from the original biological fluid, the analyte is ready to assay. An advantage of the solution step is that it permits the analyte to be assayed by a homogeneous method such methods being generally preferable in terms of cost, speed, simplicity and robustness to heterogeneous methods. The method of the invention is particularly well adapted to the case where the analyte (or fragment) is assayed by an agglutination technique. The extent of agglutination may be determined by turbidimetry, but is preferably determined by a particle counting technique such as our IMPACT (Trade Mark) diagnostic technique. This system involves counting particle numbers in a defined volume of liquid. The particles are of known uniform size, and the instrument is calibrated to count only particles of approximately that size, so that assemblies of two or more particles formed by agglutination are rejected and not counted.

In this preferred method according to the invention, the specific binder used in step a) has previously been insolubilised by attachment to first particles of a particular size (size A). The particles, carrying an insolubilised analyte/specific binder complex are separated from the reaction mixture, washed and then treated with enzyme to re-solubilise the analyte. The solution containing the analyte is then assayed by a known agglutination technique using coated second particles of a different size (size B) when the resulting liquid, containing species in solution and also size A particles, size B particles and agglutinated size B particles, is passed to a particle counter. The latter counts only the size B particles and rejects as too large (or too small) the size A particles and the agglutinated size B particles. Alternatively, the first particles could have been made magnetically attractable and simply removed from the assay fluid at the time that agglutination of the second particles is determined.

Two preferred embodiments of this aspect of the invention will now be described.

1. This involves the use of 2.3 micron latex particles coated with antigen to extract specific antibody from a class of antibodies. This application is particularly useful for identifying the IgE antibodies directed against specific allergens in human blood serum or plasma.

In this embodiment samples are incubated with 2.3 micron latex containing the antigen (allergen) in excess covalently bound to the particles. The IgE (with specific binding sites for the allergen) binds to the particles which are then centrifuged. The supernatant is decanted to remove the serum containing the non-specific IgE molecules. The particles are resuspended in buffer, and pepsin is added to digest the allergen/specific IgE complex bound to the latex. This process releases Fc fragments of the specific IgE molecules into solution.

After quenching the reaction with neutral pH buffer, the mixture (2.3 micron particles, pepsin digest and Fc fragments) is transferred to an IMPACT or other latex agglutination/particle counting automatic analyser. A suspension of 0.8 micron latex particles with covalently bound rabbit anti-human (IgE-Fc) serum is added. Because the Fc fragments are polyvalent, latex agglutination takes place in direct proportion to the amount of Fc fragments present in the pepsin digest. The discriminators or pulse-height analyser of the instrument may be set to count unagglutinated 0.8 micron particles thus ignoring the presence of 2.3 micron particles. By the use of standards, a calibration curve may be constructed for the evaluation of test samples from patients.

Where 2.3 micron particles have been described as used in the preceding paragraphs, it will be apparent that smaller particles e.g. 0.3 microns diameter could have been used instead. By another variant, the 2.3 micron latex particles could have been made magnetically attractable. This would have enabled them to be pulled out of suspension in the presence of the 0.8 microm particles, and would have permitted a turbidometric method instead of a particle counting one.

As an assay for a specific IgE, this method has advantages over that described in EPA 51985. First, because the IgE is usually a minor component of the immunoglobulin content of the sample most or all of which may be immunoreactive with the allergen, a large surface area coated with allergen is required to ensure complete IgE binding. Such a surface area may be more than can easily be provided by an absorbent paper disc, but is easily achieved by the use of a suitable volume of first particles coated with allergen. Second, when the allergen/IgE complex is treated with enzyme to solubilise the IgE or fragment, the first particles can simply be left in the assay fluid, no separation step being necessary at this stage.

2. This involves the use of 2.3 micron latex particles coated with antibody to extract free antigen from serum containing also protein-bound antigen.

This application is particularly useful for the determination of the non-protein-bound portion of small molecules (such as thyroxine ($T_4$), triiodothyronine ($T_3$), cortisol, testosterone and drugs such as diphenylhydantoin, sodium valproate, and carbamazepine). These substances are transported in blood reversibly bound to carrier proteins. In the case of $T_4$ 99.98% is bound to three proteins in serum, namely

thyroxine binding globulin, prealbumin and serum albumin. Similarly 99.8% of $T_3$ is bound to these three proteins. Approximately 90% of cortisol is bound to serum proteins (transcortin and albumin). In the case of testosterone, about 98% is bound to two proteins (sex hormone binding globulin and albumin). The three drugs mentioned are used in anti-convulsant therapy and 95% of each of these drugs is bound to albumin in serum. In all cases, it is the free analyte concentration that correlates best with clinical status. Hence a measurement of the free portion without interference from the much greater protein-bound portion is a very useful tool for aiding diagnosis and patient management.

The technique requires the use of latex coated with an antibody specific for the analyte to be measured. The amount of antibody is generally in excess of the expected amount of free analyte, but should be insufficient to substantially perturb the equilibrium between protein-bound and free forms. It is particularly important that the amount of antibody be the same from one assay tube to the next. As in the earlier embodiment, the latex particle size used for this extraction step a) should be larger (or smaller) than the latex particles used in the second stage of the assay.

The first latex is used to extract a quantity of analyte proportional to the free analyte concentration as follows:-

The free analyte concentration (F) for an analyte of total concentration (T) in equilibrium with bound forms bound to a series of i proteins with total protein concentrations Pi (where i = 1 to n), is given by the following equation:-

$$T = F\left[1 + \sum_{i=1}^{i=n} \frac{K_i P_i}{1 + K_i F}\right] \qquad (1)$$

Using latex antibody reagent - 1 to extract a small portion of analyte, the equation becomes:-

$$T = F_i\left[1 + \sum_{i=1}^{i=n} \frac{K_i P_i}{1 + K_i F'} + \frac{K_{n+1} P_{n+1}}{1 + K_{n+1} F'}\right] \qquad (2)$$

The amount of the antibody (Pn+1) and affinity (Kn+1) should be such that the following conditions are held true:-

$$F' \triangleq F$$

and

$$\frac{K_{n+1} P_{n+1}}{1 + k_{n+1} F'} \ll \left[1 + \sum_{i=1}^{i=n} \frac{K_i P_i}{1 + K_i F'}\right]$$

In many respects a low affinity antibody such as a monoclonal antibody is particularly useful because the amount of analyte extracted is directly proportional to free analyte concentration over a wide range:-

$$\text{Analyte extracted} \triangleq K_{n+1} P_{n+1} F'$$

Whereas for high affinity antibodies:-

$$\text{Analyte extracted} \triangleq \left[ \frac{K_{n+1} \; P_{n+1} \; F'}{1 + K_{n+1} \; F'} \right]$$

Under these circumstances, at high free analyte concentrations a limiting value is obtained (equivalent to antibody capacity Pn + 1) and is thus independent of free analyte concentration.

The method involves using a latex first antibody reagent to extract the analyte in a short first incubation step. The mixture is centrifuged, the supernatant decanted and pepsin (HCl) is added to the precipitate to release analyte from the latex reagent. The analyte in solution is then assayed, e.g. by techniques described in US Patent 4184849 or 4427781. Use is made of a second latex with a particle size suitable for use in the particle size analyser (counter). As before, the larger (or smaller) inactivated first antibody latex is not registered by the counter and the latex agglutination reaction of the second antibody latex is analysed by the counter as per the IMPACT system. The method is superior to that described in GB 2030290A (discussed above) because it is faster and also drift-free. The use of first latex particles coated with antibody permits accurate control of the amount of antibody presented to sample, such control being of critical importance, as noted above, to extract sufficient free analyte from the sample without substantially perturbing the equilibrium between protein-bound and free forms of the analyte.

The following Example illustrates the invention. The accompanying drawing is a graph of percent non-agglutinated particles against concentration of a specific IgE, determined by the following experiment.

Example

1. Reagents and buffers

The following reagents were purchased: latex particles (0.8. microns 10% W/V) with carboxyl groups (Estapor K150) and without (2.3 microns, 10% W/V, Estapor K99) from Rhone-Poulenc, Courbevoie, France; 1-ethyl-3(3-dimethyl-amino propyl) carbodiimide-HCl from Sigma Chemical, St.Louis, MO; Tween-20 from Technicon, Tarrytown, NY and bovine serum albumin (BSA) from Calbiochem, La Jolla, CA.

Buffers were borate buffered saline (BBS) with 90g/l NaCl and 20mM borate, pH 8.2; glycine buffered saline with 90g/l NaCl, 50mM glycine, pH 9.2 containing 0.1% Tween-20 (GBS-Tween) or 1% BSA (GBS-BSA) and phosphate buffered saline (PBS) with 90g/l NaCl and 8mM phosphate, pH 7.4.

2. Preparation of allergens

Glycerinated allergen extracts from Dermatophagoides pteronyssinus (DPT) and timothy pollen were purchased from Bencard Ltd (Epsom, Surrey, England). They were extensively dialysed at 4°C against PBS and concentrated by ultrafiltration on YM-10 membrane (Amicon Corp., Danvers, MA). The solutions were kept at -80°C until just before use.

3. Preparation of latex (Lx) particles

Latex of 2.3 microns diameter (Lx-2.3) was used as allergosorbent. One hundred microlitres of the 10% suspension was washed in BBS and resuspended in 200 microlitres of the same buffer; 100 microlitres was retrieved, mixed with 200 microlitres of a 10mg/ml solution of carbodiimide in water and 10 microlitres of a 10mg/ml allergen solution in BBS was added. The final volume was adjusted to 1ml with water and the suspension incubated at room temperature for 4 h while vortexing. After centrifugation at 10,000g for 10 min., Lx-2.3 was washed successively with GBS-BSA, GBS-Tween and GBS-BSA. The pellet was resuspended in 1ml of GBS-BSA and kept at 4°C. To estimate the amount of allergen present on latex, a trace amount of radiolabelled allergen was added for the incubation with latex and the residual radioactivity counted on the particles after washing. About 5 micrograms of allergen was absorbed per 100 microlitres of a 0.5% Latex suspension.

Latex of 0.8 microns diameter (Lx-0.8) was coated with Fab'2 fragments of IgG from an anti-human FcE rabbit antiserum as described by Magnusson et al., Clin. Allergy, 11, 453.

4. Assay for specific IgE

One hundred microlitres of the sample (undiluted or at a dilution of 1/5 or 1/10 in normal rabbit serum) was incubated in a conical polystyrene tube (Eppendorf) with 50 microlitres of the allergen-coated Lx-2.3 at 21°C for 90 minutes with continuous shaking. The suspension was then centrifuged for 2 minutes at 10,000 RPM in a 5414S Eppendorf Microcentrifuge, washed once in 1ml of a 90 g/l NaCl solution and finally resuspended in 50 microlitres of the same solution, 80 microlitres of 0.15 N HCl containing 1mg/ml pepsin was then added and digestion was allowed to proceed for 30 min. at 37°C before addition of 20 microlitres of 2 M Tris-NaOH.

The sample was then applied to an automatic particle counting system (IMPACT, Acade Diagnostic Systems, Brussels, Belgium) wherein 30 microlitres of the suspension was added to 30 microlitres of Lx-0.8 (0.5% suspension on GBS) coated with rabbit anti-human FcE Fab'2. Incubation was made for 30 min. at 37°C with constant shaking before counting of the non-agglutinated particles. Quantitation of IgE was made by

reference to an agglutination curve obtained by pepsin digestion of a standard amount of IgE (IgE-standard-serum, Calbiochem. La Jolla, Ca) as described Magnusson et al (see above).

Results

1. Assay Specificity

The specificity for IgF detection in this assay is based on the resistance to pepsin of a fragment of IgE called Fc" and has already been fully evaluated. In order exclude non-specific adsorption of IgE on Lx-2.3, serum samples were used with high titres of IgE but no specific IgE against DPT. Increasing amounts of a human myeloma IgE, IgE (DES), were also added to the Lx-2.3 suspension. These experiments showed that the assay was not dependent on the level of total IgE in the samples. Experiments were run with addition of soluble DPT in increasing amounts during incubation with Lx-2.3 and showed a complete inhibition of the agglutination of Lx-0.8.

2. Assay sensitivity

A typical curve is shown in the Figure. A serum sample containing a known amount of specific anti-DPT IgE as determined by immunoabsorption on insolubilised DPT was diluted twofold in PBS and mixed with Lx-2.3. The range of concentrations spread from 25 pg/ml to 200ng/ml with a maximum sensitivity between 200pg/ml and 100ng/ml. No prozone effect was seen at concentrations higher than 200ng/ml. Similar experiments were run with timothy pollen instead of DPT and gave comparable detection ranges.

The detection of serum specific IgE has been widely applied since 1972 and has greatly improved the accuracy of diagnosis of atopic disease. A radioallergosorbent test (RAST), based on the invention of US Patent 3720760 discussed above, has been favoured. This has involved coupling the allergen to a paper disc and then contacting the disc with a sample of the biological fluid to be assayed, under conditions to cause antibodies specific to the allergen to become bound to the paper disc. The disc is then washed and the extent of binding determined by means of a radiolabelled allergen or radiolabelled antibody.

This assay has however, two major drawbacks. First, the allergen is coupled to a paper disc which has a high degree of non specific protein adsorption resulting in high background values and dependence on the level of total IgE. Second, the surface of the disc being small, competition with antibodies of the same specificity but of other isotypes gives underestimation of the titre of specific IgE.

The latex agglutination assay described here has several advantages. First, the sensitivity level is much improved, reaching 0.01 IU/ml as compared to 0.7 IU/ml for the RAST. This sensitivity results from several factors: a tenfold increase of the available surface, a very low degree of non specific protein binding on latex and a reduced time of contact between the sample and the Lx-2.3. Interestingly, although the surface is greatly increased the amount of insolubilized allergen is of the same order of magnitude as on the paper disc used for the RAST (about 5 µg vs 7 µg per test). Most of the allergen, however, may not always be accessible for antibody binding in the RAST. The sensitivity of the latex assay enables detection of specific IgE in fluids where the concentration is exceedingly low like culture medium, bronchio-alveolar lavages, saliva or aqueous humour. Second, the latex assay permits a quantitative estimation of specific IgE by reference to a standard IgE preparation, unlike the RAST which results are evaluated by reference to a disc where birch pollen is insolubilized. This allows for a more precise delineation between atopics and non atopic patients.

Third, because of the increase in surface and accessibility of the allergen, competition with antibodies with the same specificity but pertaining to other isotypes (IgG, IgA and IgM) is much if not completely reduced. Serum samples containing specific anti-DPT antibodies had no residual antibody activity of any class against DPT after a single incubation with Lx-2.3. Furthermore, the latex assay can be performed in about 4 h and can be automated. It also carries the advantages inherent to the use of a pepsin digestion step before IgE detection; for example it eliminates the interferences due to auto-antibodies directed against IgE.

**Claims**

1. A method of assaying for an analyte in a biological fluid, which method comprises the steps of:-

a) contacting a sample of the biological fluid with first particles carrying a specific binder for the analyte under conditions to cause analyte to bind to the specific binder, and recovering the first particles carrying an analyte/specific binder complex,

b) treating the first particles carrying the complex to bring into solution the analyte or an antigenically distinctive fragment thereof,

c) adding second particles to the solution to form an assay fluid under conditions to effect agglutination of the second particles, and determining the extent of such agglutination as an assay of the analyte in the sample.

2. A method as claimed in claim 1, wherein the analyte is an antigen or an antibody, and the second particles carry an immune binding partner of the analyte.

3. A method as claimed in claim 1, wherein the analyte is a hapten, the second particles carry the hapten, and step c) is performed in the presence of an antibody to the hapten.

4. A method as claimed in any one of claims 1 to 3, wherein the specific binder is used in a form covalently bound to insoluble polymeric particles.

5. A method as claimed in any one of claims 1 to 3, wherein the complex is treated in step b) with an enzyme.

6. A method as claimed in any one of claims 1 to 5, wherein the first particles in step a) are of uniform size, the second particles used in step c) are of uniform size different from the first particles, and the extent of agglutination is determined in step c) by measuring the concentration of unagglutinated second particles in the assay fluid.

7. A method as claimed in any one of claims 1 to 6, wherein the analyte is a specific antibody to an allergen.

8. A method as claimed in claim 7, wherein an insolubilised antibody/specific binder complex is treated in step b) with enzyme to bring into solution Fc fragments of the antibody, which are then assayed.

9. A method as claimed in any one of claims 1 to 6, wherein the analyte is the free portion of a substance present in the biological fluid both in free form and in a form bound to one or more natural binders.

10. A method as claimed in any one of claims 1 to 9, wherein the first and second particles are latex particles.

0260079

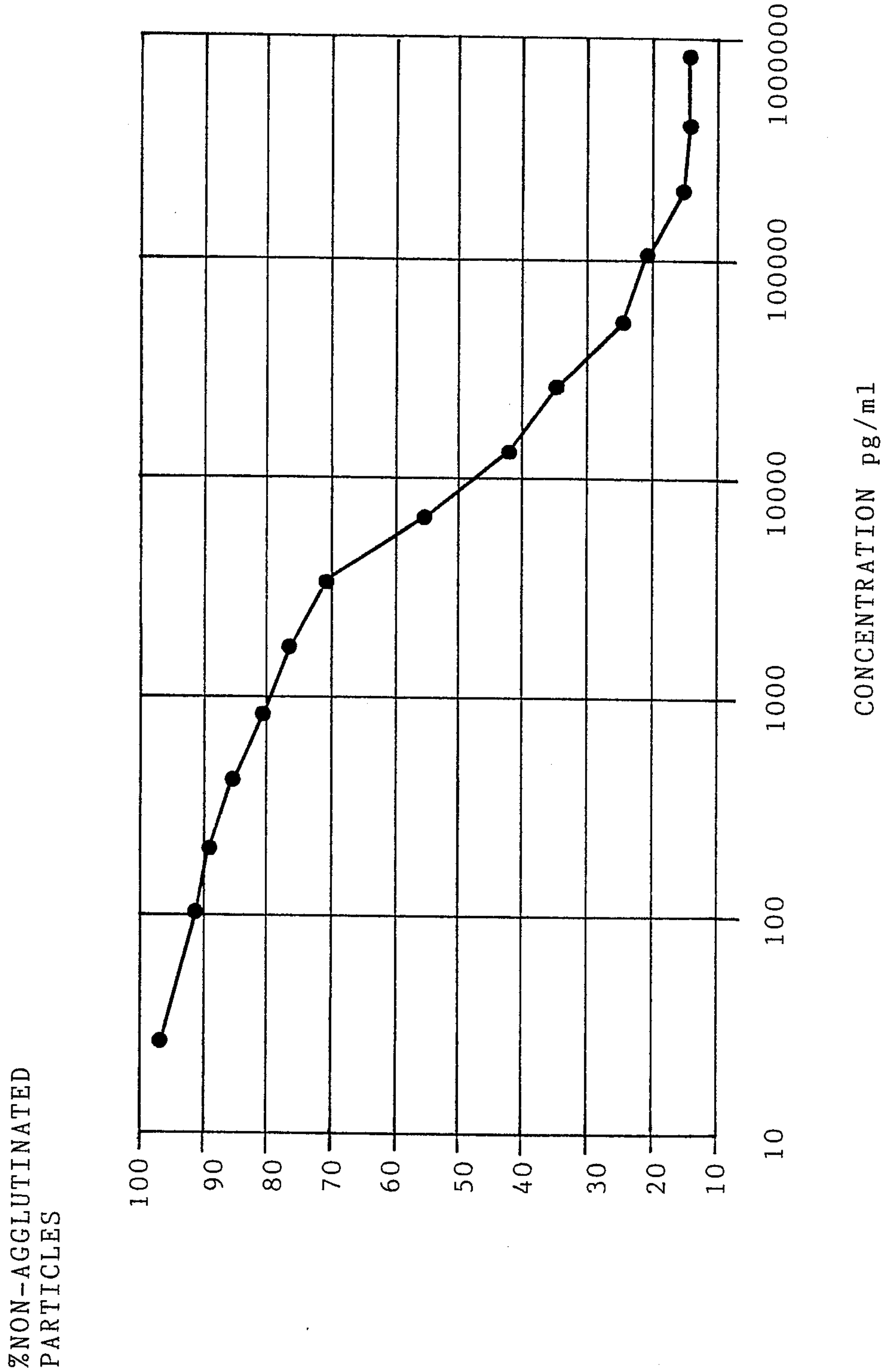
%NON-AGGLUTINATED
PARTICLES
100
90
80
70
60
50
40
30
20
10
10
100
1000
10000
100000
1000000
CONCENTRATION pg/ml

European Patent Office

# EUROPEAN SEARCH REPORT

Application number

EP 87 30 7841

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 051 985 (TECHNICON INSTRUMENTS CORP.) * Abstract; page 5, line 21 - page 6, line 18; page 7, lines 18-32; page 8, line 17 - page 11, line 19; pages 13-16, example 1; claims 7-16 * | 1-8 | G 01 N 33/546<br>G 01 N 33/563 |
| A | US-A-4 108 975 (R.H. HALES) * Abstract; claim 1 * | 1-4 | |
| A | FR-A-2 309 868 (GENERAL ELECTRIC CO.) * Claims * | 1-6 | |
| A,D | US-A-4 184 849 (C.L. CAMBIASO et al.) | | **TECHNICAL FIELDS SEARCHED (Int. Cl.4)** |
| A,D | GB-A-2 030 290 (BAXTER TRAVENOL LABORATORIES INC.) | | G 01 N |
| A | EP-A-0 019 741 (BEHRINGWERKE AG) | | |

The present search report has been drawn up for all claims

| Place of search | Date of completion of the search | Examiner |
|---|---|---|
| THE HAGUE | 11-11-1987 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document